# EUROPEAN PATENT APPLICATION

(11) **EP 4 776 455 A1**
(43) Date of publication of application: **15.07.2026**
(21) Application number: 25151153.1
(22) Date of filing: 10.01.2025
(51) Int. Cl.: H02J 1/08, H02J 7/00, H02J 7/34

(54) **NEGATIVE PRESSURE WOUND THERAPY**

(71) Applicant: Mölnlycke Health Care AB, 431 53 Mölndal (SE)
(72) Inventor: BENGTSSON, Erik, 431 41 Mölndal (SE)
(74) Representative: Zacco Sweden AB

(57) **Abstract**

An apparatus and related aspects for providing reduced pressure to a tissue site are disclosed. The apparatus comprises an electrical load and a power supply arrangement connected to the electrical load. The power supply arrangement comprises a battery, a first voltage regulator connected to the battery, a second voltage regulator connected to the first voltage regulator and to the electrical load, and a capacitor connected to the second voltage regulator. The first voltage regulator is configured to receive an input voltage from the battery and to output a supply voltage to the second voltage regulator. Moreover, the first voltage regulator is further configured to, in response to the input voltage being above a first voltage level, output the supply voltage to the second voltage regulator based on the received input voltage. The first voltage regulator is further configured to, in response to the input voltage being below a second voltage level, output a zero-voltage to the second voltage regulator. The second voltage regulator is configured to, in response to receiving the supply voltage from the first voltage regulator at an input, output a first target voltage to the load based on the received supply voltage. The second voltage regulator is further configured to, in response to receiving the zero-voltage voltage from the first voltage regulator at the input, output a second target voltage to the load by discharging the capacitor.

## Description

### TECHNICAL FIELD

The herein disclosed embodiments generally relate to negative pressure wound therapy (NPWT). In particular, the herein disclosed embodiments relate to apparatuses for providing reduced pressure to a wound site, methods for controlling an apparatus for providing reduced pressure to a wound site, and thereto related computer program products and computer-readable storage media.

### BACKGROUND

Negative pressure wound therapy (NPWT) is a technique that promotes healing of e.g. surgical, acute and chronic wounds by the application of a sub-atmospheric pressure ("negative pressure") to the wound site, using a negative pressure pump. Wound healing is achieved by applying a negative pressure, such as "vacuum" through a dressing or a cover applied onto the wound. Excess wound exudate is thereby drawn out, which increases the blood flow to the area, and promotes the formation of granulation tissue. The NPWT technique also permits less outside disturbance of the wound and transports excess fluids away from the wound site.

Portable NPWT devices are increasingly favoured due to their convenience and ability to support patient mobility during treatment. However, many of these portable devices rely on disposable batteries for power, which presents significant challenges in terms of performance and reliability.

A common issue with disposable batteries in NPWT devices is their tendency to deplete quickly, particularly when the device must handle relatively large electrical loads, such as during pump activation, valve control, loudspeaker activation, or other similar "high-power" operations. As the batteries discharge, their output voltage often drops below the levels required for the NPWT device to function effectively. This can lead to interruptions in therapy, reduced efficacy of wound treatment, and frequent battery replacements, which increase the cost and inconvenience for patients and healthcare providers.

There is therefore a need for addressing these challenges in order to improve the performance, reliability, and overall user experience of portable NPWT systems.

### SUMMARY

The herein disclosed technology seeks to mitigate, alleviate or eliminate one or more of the above-identified deficiencies and disadvantages in the prior art to address various problems relating to unstable or unreliable power supply in an NPWT system.

Various aspects and embodiments of the disclosed technology are defined below and in the accompanying independent and dependent claims.

An aspect of the disclosed technology comprises an apparatus for providing reduced pressure to a wound site. The apparatus comprises an electrical load and a power supply arrangement connected to the electrical load. The power supply arrangement comprises a battery, a first voltage regulator connected to the battery, a second voltage regulator connected to the first voltage regulator and to the electrical load, and a capacitor connected to the second voltage regulator. The first voltage regulator is configured to receive an input voltage from the battery and to output a supply voltage to the second voltage regulator. Moreover, the first voltage regulator is further configured to, in response to the input voltage being above a first voltage level, output the supply voltage to the second voltage regulator based on the received input voltage. The first voltage regulator is further configured to, in response to the input voltage being below a second voltage level, output a zero-voltage to the second voltage regulator. The second voltage regulator is configured to, in response to receiving the supply voltage from the first voltage regulator at an input, output a first target voltage to the load based on the received supply voltage. The second voltage regulator is further configured to, in response to receiving the zero-voltage voltage from the first voltage regulator at the input, output a second target voltage to the load by discharging the capacitor.

Another aspect of the disclosed technology comprises a wound treatment system comprising apparatus according to any one of the embodiments disclosed herein, a wound cover for creating a sealed space defined in part by the wound site, and a tubing assembly defining the fluid flow path. With this aspect of the disclosed technology, similar advantages and preferred features are present as in the other aspects.

Another aspect of the disclosed technology comprises a method for operating an apparatus for providing negative pressure to a wound site. The apparatus comprises an electrical load and a power supply arrangement connected to the electrical load, wherein the power supply arrangement comprises a battery, a first voltage regulator, a second voltage regulator, and a capacitor connected to the second voltage regulator. The method comprises, in response to an input voltage supplied from the battery being above a first voltage level, outputting, using the first voltage regulator, a supply voltage to the second voltage regulator based on the received input voltage, and outputting, using the second voltage regulator, a first target voltage to the electrical load based on the received supply voltage. The method further comprises, in response to the input voltage being below a second voltage level, outputting, using the first voltage regulator, a zero-voltage to the second voltage regulator, and outputting, using the second voltage regulator, a second target voltage to the electrical load by discharging the capacitor. With this aspect of the disclosed technology, similar advantages and preferred features are present as in the other aspects.

Another aspect of the disclosed technology comprises a computer program product comprising instructions which, when the program is executed by a computing device of an apparatus for providing reduced pressure to a wound dressing, causes the apparatus to carry out the method according to any one of the embodiments disclosed herein. With this aspect of the disclosed technology, similar advantages and preferred features are present as in the other aspects.

Another aspect of the disclosed technology comprises a (non-transitory) computer-readable storage medium comprising instructions which, when executed by a computing device of an apparatus for providing reduced pressure to a wound dressing, causes the apparatus to carry out the method according to any one of the embodiments disclosed herein. With this aspect of the disclosed technology, similar advantages and preferred features are present as in the other aspects.

The term "non-transitory," as used herein, is intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link. Thus, the term "non-transitory", as used herein, is a limitation of the medium itself (i.e., tangible, not a signal) as opposed to a limitation on data storage persistency (e.g., RAM vs. ROM).

The disclosed aspects and preferred embodiments may be suitably combined with each other in any manner apparent to anyone of ordinary skill in the art, such that one or more features or embodiments disclosed in relation to one aspect may also be considered to be disclosed in relation to another aspect or embodiment of another aspect.

An advantage of some embodiments is that the useable lifetime of batteries for NPWT devices may be improved, thereby reducing the environmental footprint of NPWT devices.

An advantage of some embodiments is that the NPWT devices may be provided with improved redundancy and capable of providing important functionalities even when the battery voltage output is temporarily reduced due to voltage sag.

An advantage of some embodiments is that the NPWT device may be more cost efficient with maintained performance as a wider variety of batteries can be used without drastically impairing operational lifetime.

An advantage of some embodiments is that the carbon footprint of the NPWT devices may be reduced as one can use batteries with a lower energy density but smaller carbon footprint in terms of resource extraction and manufacturing, such as alkaline batteries instead of lithium batteries without drastically impairing performance and reliability if the devices.

An advantage of some embodiments is that NPWT devices may be rendered more environmentally sustainable since the negative effects associated with the degradation of rechargeable batteries over time may at least partly be mitigated, enabling the use of such batteries for longer periods of time.

Further embodiments are defined in the dependent claims. It should be emphasized that the term "comprises/comprising" when used in this specification is taken to specify the presence of stated features, integers, steps, or components. It does not preclude the presence or addition of one or more other features, integers, steps, components, or groups thereof.

These and other features and advantages of the disclosed technology will in the following be further clarified with reference to the embodiments described hereinafter.

### BRIEF DESCRIPTION OF THE DRAWINGS

The above aspects, features and advantages of the disclosed technology, will be more fully appreciated by reference to the following illustrative and non-limiting detailed description of example embodiments of the present disclosure, when taken in conjunction with the accompanying drawings, in which:
Fig. 1 is a schematic illustration of a wound treatment system in accordance with some embodiments.
Fig. 2 is a schematic illustration of a wound treatment system in accordance with some embodiments.
Fig. 3 is a schematic block diagram representation of an apparatus for providing negative pressure to a wound site in accordance with some embodiments.
Fig. 4 is a schematic flowchart representation of a method for operating an apparatus for providing negative pressure to a wound site in accordance with some embodiments.

### DETAILED DESCRIPTION

The present disclosure will now be described in detail with reference to the accompanying drawings, in which some example embodiments of the disclosed technology are shown. The disclosed technology may, however, be embodied in other forms and should not be construed as limited to the disclosed example embodiments. The disclosed example embodiments are provided to fully convey the scope of the disclosed technology to the skilled person. Like reference characters refer to like elements throughout. Those skilled in the art will appreciate that the steps, services and functions explained herein may be implemented using individual hardware circuitry, using software functioning in conjunction with hardware circuitry such as a programmed microprocessor or general-purpose computer, using one or more Application Specific Integrated Circuits (ASICs), using one or more Field Programmable Gate Arrays (FPGA) and/or using one or more Digital Signal Processors (DSPs).

It will also be appreciated that when the present disclosure is described in terms of a method, it may also be embodied in an apparatus comprising one or more processors, one or more memories coupled to the one or more processors, where computer code is loaded to implement the method. For example, the one or more memories may store one or more computer programs that cause the apparatus to perform the steps, services and functions disclosed herein when executed by the one or more processors in some embodiments.

It is also to be understood that the terminology used herein is for purpose of describing particular embodiments only, and is not intended to be limiting. It should be noted that, as used in the specification and the appended claim, the articles "a", "an", "the", and "said" are intended to mean that there are one or more of the elements unless the context clearly dictates otherwise. Thus, for example, reference to "a unit" or "the unit" may refer to more than one unit in some contexts, and the like. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative. Similarly, "at least one of A and B" is to be interpreted as only A, only B, or both A and B.

It will also be understood that, although the term first, second, etc. may be used herein to describe various elements or features, these elements should not be limited by these terms. These terms are only used to distinguish one element from another. For example, a first signal could be termed a second signal, and, similarly, a second signal could be termed a first signal, without departing from the scope of the embodiments. The term "operatively connected" is in the context of the present disclosure to be understood as that the "operatively connected" entities or units can transmit and/or receive signals to and/or from each other.

As used herein, the term "in response to" may be construed to mean "when or "upon" or "if" depending on the context. Similarly, the phrase "if it is determined' or "when it is determined" or "in an instance of" may be construed to mean "upon determining or "in response to determining" or "upon detecting and identifying occurrence of an event" or "in response to detecting occurrence of an event" depending on the context. Accordingly, the phrase "if X equals Y" may be construed as "when X equals Y", "when it is determined that X equals Y", "in response to X being equal to Y", or "in response to detecting/determining that X equals Y" depending on the context.

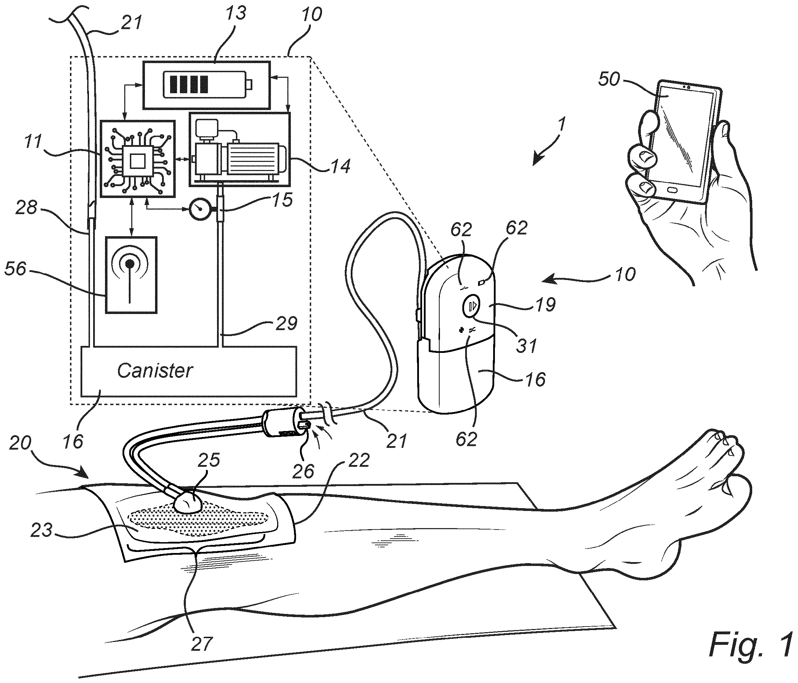

Turning now to the drawings, and to Fig. 1 in particular, there is schematically illustrated a wound treatment system 1 (sometimes simply referred to as "the system" 1) in accordance with some embodiments. The depicted wound treatment system 1 may be referred to as an "NPWT system" 1, "reduced pressure wound treatment system" 1, or "negative pressure wound treatment system" 1. The wound treatment system 1 comprises an apparatus 10 for providing reduced pressure to a wound site 27 (or otherwise referred to as a tissue site 27), a wound cover 22 for creating a sealed space defined in part by the wound site 27, and a tubing assembly fluidly connecting the apparatus 10 to the wound site 27.

Accordingly, the wound cover 22 is adapted to create a sealed space 23 defined in part by a wound surface, such as at the skin of a user/person, at or around a wound of the user/person. Further, the apparatus 10 ("NPWT device") is fluidly connected to the wound cover 22 using e.g. a tubing assembly 21 ("tubing"). The tubing 21 may be of any suitable flexible tubing fabricated from elastomeric or polymeric materials. The tubing 21 may connect to the wound dressing 20, or more specifically to the wound cover 22 of the wound dressing 20, via a suitable connector 25. The connector 25 may be adhered or otherwise attached to the wound cover 22. In particular, the connector 25 may be attached around an opening (not shown) formed in the wound cover 22. The tubing 21 may comprise one, two, or more individual tubes.

The expression "wound cover" as used herein should be interpreted broadly as any wound site member, e.g. it can be a film sealed around a periphery of a wound site 27, where a wound filler may be used to fill the wound volume prior to the application of such wound cover. Wound cover may also refer to a backing layer of a wound dressing 20 comprising an additional layer(s) such as for example an absorbent layer and/or a spacer layer.

The apparatus 10 comprises a source of negative pressure 14 configured to provide negative pressure (i.e. sub-atmospheric pressure) via a fluid flow path from an inlet of the source of negative pressure 14 to the wound dressing 20, or more specifically to provide negative pressure under a wound cover. In some embodiments, the source of negative pressure 14 comprises a negative pressure pump that together with a motor is adapted for establishing a negative pressure when the source of negative pressure 14 is operating, i.e. when it is in an active state or simply "active". The source of negative pressure may comprise any type of pump and motor that are biocompatible or otherwise suitable for use in an NPWT setting and capable of maintaining or drawing adequate and therapeutic vacuum levels. Preferably, the negative pressure level to be achieved is in a range between about -20 mmHg and about -300 mmHg. In some embodiments, a negative pressure range between about -80 mmHg and about - 140 mmHg is used. In some embodiments, a negative pressure range between about -115 mmHg and about -135 mmHg is used. In some embodiments, the negative pressure pump is a diaphragm pump, a peristaltic pump, piezoelectric pump or the like, in which a motor causes the moving parts to draw fluid from the wound site 27.

In the context of the present disclosure, it should be understood that the expressions "negative pressure", "sub-atmospheric pressure", "reduced pressure", or even "vacuum", as used interchangeably herein, generally refer to a pressure less than a local ambient pressure, such as the ambient pressure in a local environment external to a sealed therapeutic environment provided by a wound cover 22. In many cases, the local ambient pressure may also be the atmospheric pressure at which a patient is located. Thus, the "negative pressure" may be understood as a pressure difference between the ambient pressure and the pressure under the wound cover, where ambient pressure is generally set as 0 mmHg. Unless otherwise indicated, values of pressure stated herein are gauge pressures. Similarly, references to increases in negative pressure typically refer to a decrease in (absolute) pressure, while decreases in negative pressure typically refer to an increase in (absolute) pressure.

Further, in some embodiments the apparatus 10 comprises a canister 16 fluidly connected to the negative pressure source 14. The canister 16 may be formed from e.g. moulded plastic or the like, and possibly be a detachable component of the apparatus 10. Moreover, the canister 16 may further be at least partly transparent/translucent to permit viewing into the interior of the canister 16 to assist the user in determining the remaining capacity of the canister 16. As used herein, the term "fluidly connected" should be interpreted broadly and may comprise e.g. any form of tubing, conduits, or channels providing a fluid connection/communication between the canister 16 and the negative pressure source 14 and the dressing 20.

In some embodiments, the canister 16 comprises an inlet port 28 for allowing connection to the tubing 21. The inlet port 28 may also be formed elsewhere at the apparatus 10, however still fluidly connected to the canister 16. The connection between the inlet port 28 and the tubing 21 is a sealed connection, thus ensuring that no leakage is formed at the inlet port 28 during normal operation of the apparatus 10. The tubing 21 is preferably detachably connected to the inlet port 28 through conventional means including a friction fit, bayonet coupling, snap fit, barbed connector, or the like. The inlet port 28 may be moulded/formed from the same material and/or at the same time as forming the canister 16. A similar sealed connection (e.g. using a flange insulation/"O-ring") is formed between the canister 16 (at the outlet port 29) and the source of negative pressure 14. The canister 16 may form a part of the first fluid flow path.

In some embodiments, the apparatus comprises a housing 19 enclosing the negative pressure source 14. The canister 16 may be detachably connected to a housing 19 comprising the negative pressure source 14, whereby e.g. a full canister may be removed and replaced with an empty (new) canister. In such embodiments it may be desirable to provide e.g. the canister 16 and the housing 19 with some form of engagement means for securing the canister 16 to the housing such that the canister 16 is not unintentionally removed from the housing 19. The engagement means may in one embodiment comprise a pair of flexible protrusions extending from the canister 16 and adapted to engage with e.g. corresponding locking grooves provided at the housing 19.

However, in some embodiments, the wound treatment system 1 is a canister-less wound treatment system (not shown), where the wound exudate is collected in an absorbent layer, or the like, of the wound dressing 20. In such embodiments, the fluid flow path from the wound dressing 20 to the negative pressure source 14 is provided with one or more suitable filters (not shown) that are adapted to allow gas flow from the wound site 27 to the source of negative pressure 14 and block the flow of liquids from the wound site 27 to the source of negative pressure 14, as readily understood by the person skilled in the art.

The apparatus 10 further comprises control circuitry 11 (may also be referred to as "a control unit", "a controller", or the like) electrically connected to a power supply 13 and the source of negative pressure 14. The control circuitry 11 is configured to control operation of the source of negative pressure 14. The control circuitry 11 may comprise a microprocessor, microcontroller, programmable digital signal processor or another programmable device. The control circuitry 11 may also, or instead, include an application specific integrated circuit (ASIC), a programmable gate array or programmable array logic, a programmable logic device, or a digital signal processor. Where the control circuitry 11 includes a programmable device such as the microprocessor, microcontroller or programmable digital signal processor mentioned above, the control circuitry 11 may further include computer executable code that controls operation of the programmable device. Thus, in some embodiments, the control circuitry 11 comprises one or more processors and a memory, where the memory contains instructions executable by the processor whereby the apparatus 10 is operative to execute any one of the method steps or functions disclosed herein.

In some embodiments, the apparatus 10 comprises power supply arrangement 13 comprising a power source, such as e.g. a battery for powering the apparatus 10. The battery may be of the rechargeable type but may alternatively be arranged to be disposable and thus to be changed once discharged. A specifically adapted battery pack may be used in relation to some embodiments. The apparatus 10 may be of single-use type (allowing use during one single treatment time duration) or multiple-use type (allowing use during several different treatment sessions). Moreover, if employing batteries of the rechargeable type, it is known that these batteries may degrade over time and after a number of recharge cycles. Thus, an advantage of some embodiments is that the negative effects associated with the degradation of rechargeable batteries over time may at least partly be mitigated, enabling the use of such batteries for longer periods of time.

The power supply arrangement 13 (or "power supply circuitry") may further comprise suitable components to regulate the supply voltage that is provided to various components (or "electrical loads") of the apparatus 10, such as to the source of negative pressure 14, the general control circuitry 11, and so forth. For example, the power supply arrangement 13 may comprise one or more voltage regulators/converters (e.g., boost regulators or buck-boost regulators). However, in some embodiments, the voltage regulators/converters are provided as a part of the control circuitry 11 depending on requirements. However, in cases where the voltage regulators/converters are provided as a separate component to the control circuitry 11, they are still operatively connected to the control circuitry 11 and may therefore controlled by the control circuitry 11.

Furthermore, in some embodiments, the apparatus 10 comprises one or more pressure sensors 15 arranged in fluid connection with the inlet of the source of negative pressure 14, the canister 16, and/or the sealed space 23. In the depicted embodiment of Fig. 1, the apparatus 10 comprises a pressure sensor 15 arranged in a fluid flow path between the canister 16 and the source of negative pressure 14. However, as readily understood by a person skilled in the art, the pressure sensor 15 may be located at any other suitable location to sense or detect a pressure within the fluid flow path between the source of negative pressure 14 and the wound site. For example, the pressure sensor 15 may be arranged within the canister or under the wound cover 22.

During use of the apparatus 10, the wound cover 22 is arranged at a wound site 27 of the user/patient, forming the sealed space 23. A tubing assembly is provided with a first tubing 21 to fluidly connect the wound cover 22 to the inlet port 28 of the apparatus 10. The apparatus 10 is then activated, e.g. by a user pressing a start/pause button 31 of a user-interface of the apparatus 10 that activates the source of negative pressure 14. Alternatively, activation may be initiated via an external device 50 connected to the system 1. The apparatus 10 may for example comprise a suitable communication interface 56 to wirelessly connect to the external device 50.

When activated, the source of negative pressure 14 will start to evacuate air through the canister 16, the inlet port 28, the tubing 21 and the sealed space 23 formed by the wound cover 22. Accordingly, a negative pressure will be created within the sealed space 23. This initial evacuation of air following a start-up of the apparatus 10 may be referred to as "depressurization". The initial evacuation of air (i.e., "depressurization") may continue until reaching a set pressure value (e.g., -125 mmHg). In some embodiments, the control circuitry 11 is configured to control the source of negative pressure 14 so to establish and maintain a negative pressure level (as measured by the pressure sensor 15) within a negative pressure range. The negative pressure range may for example be -20 mmHg to -300 mmHg, - 80 to -180 mmHg, -100 to -150 mmHg, -110 to -140 mmHg, -115 to -135 mmHg, or any suitable subrange thereof (e.g., -80 mmHg to -100 mmHg). The negative pressure range may for example be selected in view of the type of wound and/or the patient.

In case a liquid has been formed at the wound site 27, this liquid from the wound site 27 may at least partly be "drawn" from the wound site, through the tubing 21, the inlet port 28 and into the canister 16. The amount of liquid (possibly defined as exudate) that is drawn from the wound and collected in the canister 16 will depend on the type of wound that is being treated, the duration of the treatment, as well as the type of wound dressing 20 used. For example, in case an absorbent dressing is used, the liquid may be absorbed and collected both in the canister 16 and the wound dressing 20, whereas if a dressing with no absorption capacity or little absorption capacity is used, most or all of the liquid from the wound site 27 may be collected in the canister 16. A suitable filter member (not shown) is generally arranged in proximity to the outlet port 29 of the canister 16 to ensure that no liquid is allowed to pass to the source of negative pressure 14 from the canister 16.

In some embodiments, the wound treatment system 1 further comprise an air inlet port 26 or a controlled leakage port 26 that provides a small and controlled inflow of air (as indicated by the arrows) under the wound cover 22. The air inlet port 26 may for example be provided via a secondary lumen extending from the connector 25 as schematically illustrated. However, in some embodiments the air inlet port 26 may additionally or alternatively be provided at the connector 25 and/or along any suitable location of the tubing 21.

Accordingly, the wound treatment system 1 may in some embodiments be arranged to ensure that a small controlled leakage is present, ensuring that a flow from the sealed space 23 may be achieved (under the assumption that there is no general blocking e.g. within the tubing 21). Additionally, during use of the wound treatment system 1 some leakage may be expected in relation to the wound cover 22. In some embodiments, the air inlet port 26 includes means for supplying/providing air to the wound site 27 at a predetermined supply rate, the rate being from 2 to 7 ml/min, preferably from 3 to 5 ml/min at a specified level of negative pressure. The predetermined level of negative pressure may be a value within the range from -80 to -180 mmHg, preferably from -100 to -150 mmHg, more preferably from - 110 to -140 mmHg, from -115 to -135 mmHg, or -120 to -135 mmHg. The means for providing air to the wound site 27 (i.e. under the wound cover 22) at a predetermined supply rate may for example be in the form of an air filter (not shown).

In some embodiments, the air filter comprises a hydrophobic and porous material, where the size of the pores is within the range of from 2 to 20 µm, preferably in the range of from 5 to 12 µm. The pore size of the filter is measured in a non-compressed state. In some embodiments, the air filter comprises polyethylene or sintered polyethylene.

As mentioned, the apparatus 10 may comprise a communication interface 56. The communication interface 56 may comprise suitable components (e.g. transceivers, antennas, filters, power amplifiers, etc.) with suitable communication protocols (e.g., Wi-Fi, Bluetooth, Bluetooth Low Energy, Zigbee, or the like) in order to establish a connection with an external device 50 and to transmit and receive wireless signals to and from the external device 50. The communication interface 56 is operatively connected to the control circuitry 11. The communication interface 56 may be configured to transmit and receive wireless signals to and from an external handheld device 50. In more detail, the communication interface 56 comprises one or more transceivers and one or more antennas connected to the one or more transceivers. The one or more transceivers are accordingly configured to transmit signals to an external device 50 via the one or more antennas and to receive signals from an external device 50 via the one or more antennas. For example, the communications interface 56 may include a Wi-Fi transceiver for communicating via a wireless communications network, cellular/mobile phone communications transceiver. In another example, the communication interface 56 may include a short-range wireless transceiver (e.g., a Bluetooth wireless transceiver, etc.) for communicating via a short-range wireless communication network.

The external device may be an external handheld device 50, such as a mobile phone (e.g. a smart phone) operatively connected to the apparatus 10.

Still further, the apparatus 10 may comprise one or more Light Emitting Diodes (LEDs) 62. The one or more LEDs 62 may be considered to form a User Interface (Ul) together with the button 31 arranged on the housing 19. The LEDs 62 may for example be arranged on the housing 19 of the apparatus 10. The LEDs 62 may be arranged under partly transparent portions of the housing to indicate various operating conditions of the apparatus 10. In more detail, the LEDs may be used to indicate a State of Charge (SoC) or available capacity of the power supply arrangement 13, presence of a blockage condition, and/or a presence of a leakage condition. The aforementioned transparent portions may accordingly be formed as suitable elements/icons indicating the various operating conditions of the apparatus 10 (e.g., a battery-shaped element/icon for indicating battery capacity). Moreover, each LED may be arranged to emit light of different colours (e.g., green, yellow, red) to provide more granularity in the indications. For example, a green battery icon may indicate high battery capacity (e.g., SoC > 50%), a yellow battery icon may indicate a moderate battery capacity (e.g., 50% > SoC > 10%), while a red battery icon may indicate a low battery capacity (e.g., SoC < 10%). The apparatus 10 may comprise further LEDs than those indicated in the drawings. For example, the apparatus 10 may comprise an LED associated with the button 31 in order to indicate an active state of the apparatus 10.

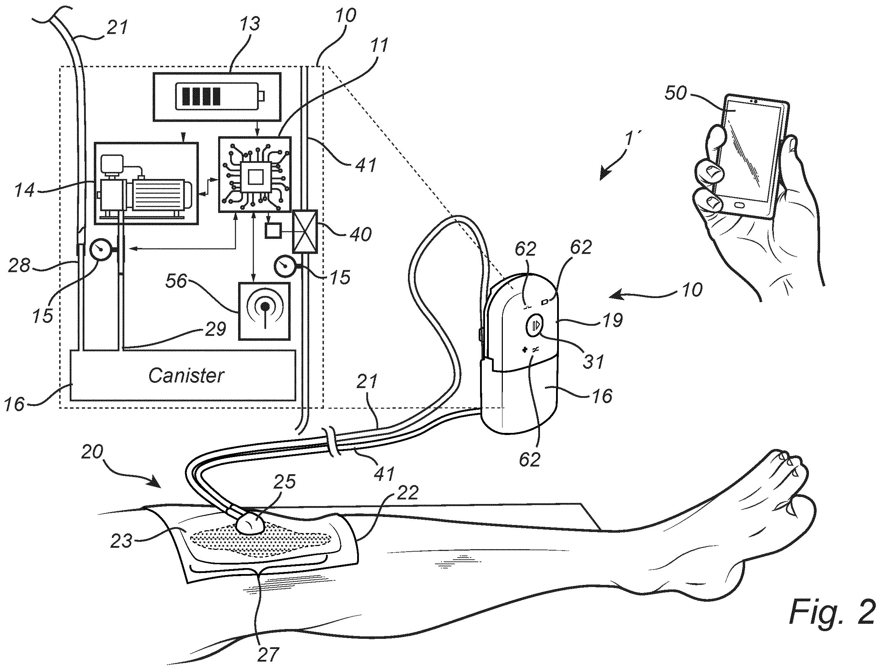

Fig. 2 depicts a wound treatment system 1' in accordance with some embodiments. Many functions, features and components of the wound treatment system 1' depicted in Fig. 2 are the same as or analogous to the wound treatment system 1 depicted in Fig. 1 and will for the sake of brevity and conciseness not be repeated. Instead, the focus will be on the differentiating functions, features, or components between the two wound treatment systems. Thus, as the person skilled in the art readily understands, unless specifically indicated, the foregoing discussion related to the components of the wound treatment system 1 with reference to Fig. 1 is applicable for the corresponding components depicted in Fig. 2 as indicated by the reference numerals.

In general, the wound treatment system 1 depicted in Fig. 1 differs from the wound treatment system 1' depicted in Fig. 2 in that the former is often referred to as a "single-lumen" NPWT system 1 while the latter is often referred to as a "dual-lumen" NPWT system 1'. Accordingly, while the wound treatment system 1 depicted in Fig. 1 has a "controlled inflow of air" via leakage (also referred to as a controlled leakage), the wound treatment system 1' depicted in Fig. 2 does not have a controlled leakage, but instead explicitly controls or regulates the air/fluid supply to the wound site 27 by opening and closing an electronically controllable valve 40 ("valve").

Therefore, in contrast to the wound treatment system 1 depicted in Fig. 1, the wound treatment system 1' of Fig. 2 comprises a second fluid flow path that fluidly connects the sealed space 23 created by the wound cover 22 to the ambient atmosphere or a "fluid reservoir" (not shown) via an electronically controllable valve 40. The second fluid flow path (may also be referred to as "air lumen") is defined by a second tubing 41 that may be of any suitable flexible tubing fabricated from elastomeric or polymeric materials. Here, the fluid flow path from the inlet of the source of negative pressure 14 to the wound dressing 20 may be construed as a "first" fluid flow path or "exudate lumen".

Here, the control circuitry 11 is electronically connected to the electronically controllable valve 40, and the control circuitry 11 is further configured to control an operation of the electronically controllable vale 40 so to release negative pressure from the wound site via the second fluid flow path (or "air lumen") 41. In other words, the control circuitry 11 is configured to open the electronically controllable valve 40 so to introduce fluid (e.g. air) to the wound site 27 when there is a sub-atmospheric pressure under the wound cover 22. This operation may also be referred to as "flushing".

Since the wound treatment system 1' depicted in Fig. 2 does not have any "controlled leakage flow", there is a risk that the apparatus 10 would not be able to draw any, or at least a sub-optimal amount of "exudate" from the wound site 27 to the canister 16 once negative pressure has been established under the wound cover 22 due to lack of airflow through the system 1'. Therefore, in order to be able to draw desired amounts of wound exudate from the wound site, a fluid, such as e.g. air from the ambient atmosphere, may be controllably introduced at defined time intervals and/or in response to pressure measurements by opening the electronically controllable valve 40. Once enough fluid has been introduced (e.g. in response to a negative pressure within the first and/or second fluid flow path reaching a lower threshold), the control circuitry 11 is configured to close the electronically controllable valve 40 and to activate the source of negative pressure 14.

The ability to controllably "flush" the system 1' by injecting air via an electronically controllable valve 40 may provide the advantage of longer pressure regulation cycles, which reduces the on-time for the source of negative pressure 14 and therefore reduces the energy consumption of the apparatus 10.

As before, the apparatus 10 of the wound treatment system 1' may comprise one or more pressure sensors 15 arranged in fluid connection with the inlet of the source of negative pressure 14, the canister 16, and/or the sealed space 23. However, the apparatus 10 may alternatively or additionally comprise a pressure sensor 15 arranged in fluid connection with the second fluid flow path (i.e., "air lumen"). The pressure sensor 15 (i.e., "air lumen pressure sensor") is preferably arranged to measure a pressure level in the second fluid flow path downstream of the electronically controllable value 40 (i.e., between the valve 40 and the wound site 27). In the depicted embodiment of Fig. 2, the apparatus comprises two pressure sensors 15 a first pressure sensor 15 configured to measure a pressure level within the first fluid flow path, and a second pressure sensor 15 configured to measure a pressure level within the second fluid flow path. However, the apparatus 10 may comprise a single pressure sensor arranged in any one of the suitable locations mentioned in the foregoing.

The apparatus 10 may further comprise an air filter (not shown) arranged in the second fluid flow path in order to limit the airflow when the electronically controllable valve 40 is open. In some embodiments, the air filter comprises a hydrophobic and porous material, where the size of the pores is configured to allow for a flow in the range of 50-120 ml/s, such as 60-100 ml/s when the valve 40 is opened after negative pressure has been established under the wound cover 20. In other words, the size of the pores is configured to allow for a flow in the range of 50-120 ml/s, such as 60-100 ml/s when the valve 40 is opened and when there is a specific negative pressure level within the system 1'. The pore size of the filter is preferably measured in a non-compressed state. In some embodiments, the air filter comprises polyethylene or sintered polyethylene. The air filter may also serve to reduce the risk of contaminants entering the wound site 27 when the valve 40 is opened (i.e., during "flushing"). The air filter may be arranged in the tube 41 that at least partly defines the second fluid flow path. The air filter may be arranged upstream of the valve 40 (i.e., between the valve 40 and the inlet of ambient air, or downstream of the valve 40 such as between the valve and the air lumen pressure sensor 15.

Moving on, regardless of the wound treatment system 1, 1' being single lumen (Fig. 1) or dual lumen (Fig. 2), the apparatus 10 comprises at least one electrical load and a power supply arrangement 13 connected to the electrical load. The electrical load may for example be the source of negative pressure 14, the electronically controllable valve 40, a loudspeaker 84, the communication interface 56 or any other subcomponent thereof, a Low-dropout regulator (LDO) 87, or the general control circuitry 11 ("control unit").

In reference to the LDO 87, the apparatus 10 may comprise an LDO 87 connected between the power supply arrangement 13 and the pressure sensor 15. The LDO 87 plays a role in ensuring reliable operation of the pressure sensors 15. In more detail, pressure sensors 15 often require a stable and precise voltage for accurate measurements. Fluctuations in the input power source can lead to errors in sensor readings. The LDO ensures a consistent output voltage to the pressure sensors, regardless of variations in the input voltage (e.g., from the power supply arrangement 13).

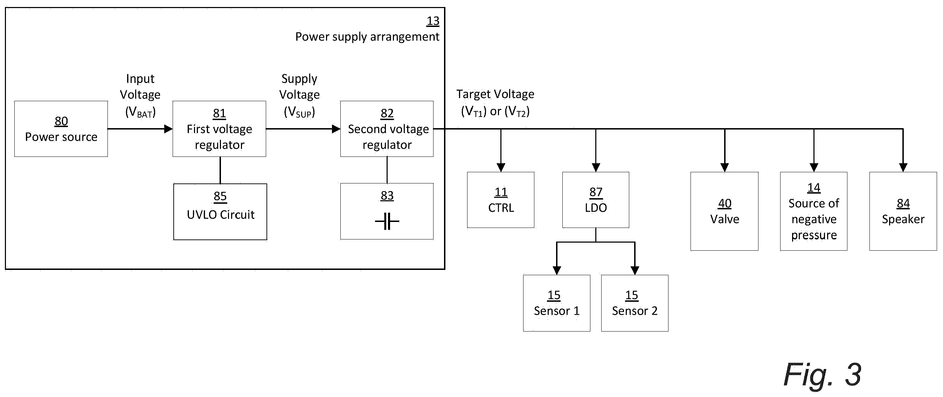

Turning now to Fig. 3, to better elucidate some embodiments herein, there is provided a schematic block diagram representation of an apparatus 10 for providing reduced pressure to a wound site 27, in accordance with some embodiments. As mentioned, the apparatus 10 comprises an electrical load and a power supply arrangement 13 connected to the electrical load. The power supply arrangement 13 comprises a power source 80, a first voltage regulator 81 connected to the power source 80, a second voltage regulator 82 connected to the first voltage regulator 81 and to the electrical load, and a capacitor 83 connected to the second voltage regulator 82. The power source 80 is preferably an electro-chemical power source, such as a battery 80.

The battery 80 outputs a battery voltage (V_{BAT}), referred to as "input voltage" herein. The input voltage (V_{BAT}) will generally vary within some tolerance levels depending on the current draw from the various components ("electrical loads") of the apparatus. In more detail, the momentary drop in a battery's output voltage when it is connected to a load is typically caused by the internal resistance of the battery and the sudden demand for current. Key factors contributing to this voltage drop include internal resistance, initial current surge, electrochemical lag, temperature effects, and battery state of charge (SoC).

Every battery has some inherent internal resistance due to the materials and chemical processes involved. When a load is connected, current flows through the battery, and a voltage drop occurs across its internal resistance according to Ohm's Law (V=R*I). The larger the current demand (high electrical load), the greater the voltage drop. Moreover, when a load is first connected, there can be a sudden surge of current (initial current surge), especially in devices with capacitors, motors (e.g., source of negative pressures 14), or inductive components (e.g., electronically controllable valves 40). This sudden demand can briefly overwhelm the battery's ability to maintain a stable output voltage. Further, the chemical reactions inside the battery take a small amount of time to adjust to a sudden increase in current demand. This transient lag (electrochemical lag) in the chemical process can cause a brief drop in output voltage. Also, low temperatures can increase a battery's internal resistance, making the voltage drop more pronounced when a load is connected. Similarly, high temperatures may affect the battery's chemical activity and resistance. Lastly, a partially discharged or depleted battery is more likely to experience a significant voltage drop under load because its internal resistance tends to increase as its charge level decreases. This momentary voltage drop is typically referred to as voltage sag and can affect the performance of devices that require a stable voltage or a minimum voltage for proper operation.

To this end, in order to stabilize the output voltage from the battery 80 (V_{BAT}), one may provide a voltage regulator (may also be referred to as a "voltage converter" such as, a boost converter or a buck-boost converter) between the battery 80 and the electrical loads. A voltage regulator is a component that regulates the output voltage from the battery 80 (V_{BAT}) in order to output a required supply voltage (V_{SUP}). A voltage regulator in the form of a boost converter may be understood as a DC-to-DC converter that increases voltage, while decreasing current, from its input (supply) to its output (load). Analogously, a buck-boost converter may be understood as a type of DC-to-DC converter that has an output voltage magnitude that is either greater than or less than the input voltage magnitude.

However, voltage regulators have some limitations in their capability to increase the voltage from its input to its output. In particular, the voltage regulators may be provided with an undervoltage lockout (UVLO) function that prevents abnormal operation in the event that the input voltage (supply) is too low to ensure proper operation. The UVLO function serves to protect the downstream components from improper input voltages that may damage the components. In short, the UVLO function turns off the voltage regulator in case the input voltage is too low (i.e., below a threshold). For example, a voltage regulator in the form of a buck-boost regulator, suitable for use in an NPWT device 10, may be specified to provide a stable output voltage of 3.3 V for input voltages in the range of 1.8 V to 5 V. Should the input voltage fall below 1.8 V, then the UVLO function is configured to turn off the buck-boost regulator.

Thus, if the output voltage from the battery 80 (V_{BAT}) falls below a threshold value, the associated voltage regulator 81 is configured to output a zero-voltage (i.e., to turn off). As mentioned in the foregoing, power sources in the form of batteries may exhibit a momentary voltage drop referred to as voltage sag when particular downstream components (electrical loads) are activated, causing the voltage regulator to switch off and render the apparatus 10 unable to supply power to the downstream components. This may in particular be evident for partially depleted batteries, causing an increased need for premature battery replacement, even though the batteries are not yet completely depleted.

In order to mitigate this issue, it is herein proposed to provide the apparatus 10 with an additional voltage regulator ("second voltage regulator") 82 between the first voltage regulator 81 and the downstream components (electrical loads), and a capacitor connected to the second voltage regulator. In short, and in accordance with some embodiments herein, the second voltage regulator is configured to utilize the energy stored in the capacitor to bridge the momentary voltage drop from the battery 80 and provide the necessary voltage to the downstream components at least temporarily until the momentary voltage drop from the battery 80 has passed and power can be supplied from the battery 80 again.

In more detail, the first voltage regulator 81 is configured to receive an input voltage (V_{BAT}) from the battery 80 and to output a supply voltage (V_{SUP}) to the second voltage regulator 82. Moreover, the first voltage regulator 81 is configured to, in response to the input voltage (V_{BAT}) being above a first voltage level (V₁), output the supply voltage (V_{SUP}) to the second voltage regulator based on the received input voltage (V_{BAT}). Further, the first voltage regulator 81 is configured to, in response to the input voltage (V_{BAT}) being below a second voltage level (V₂), output a zero-voltage to the second voltage regulator. The first voltage level (V₁) and the second voltage level (V₂) may be the same value or different values. In reference to the latter, the second voltage level (V₂) may be lower than the first voltage level (V₁) to introduce a hysteresis functionality to prevent erratic operation and repeated on-off cycling of the first voltage regulator 81 when the input voltage hovers around the threshold level.

In short, the hysteresis functionality works such that when the input voltage (V_{BAT}) approaches or falls below the second voltage level (V₂), the UVLO circuit disables the voltage regulator, preventing it from operating under unsafe conditions. Without hysteresis, if the input voltage fluctuates slightly around second voltage level (V₂) (turn-off threshold), the regulator could rapidly switch on and off, which can damage components or create instability. However, with hysteresis functionality, the regulator remains off until the input voltage rises "significantly" above second voltage level (V₂), i.e., rises above the first voltage level (V₁), ensuring stable operation.

Accordingly, the first voltage regulator 81 may be configured to operate in a voltage regulation mode (i.e., buck or boost mode) in order to regulate the input voltage (V_{BAT}) in response to the input voltage (V_{BAT}) being above the first voltage level (V₁), and in a undervoltage lockout mode in order to turn off the first voltage regulator 81 in response to the input voltage (V_{BAT}) being below the second voltage level (V₂).

Further, the second voltage regulator 82 is configured to, in response to receiving the supply voltage (V_{SUP}) from the first voltage regulator 81 at an input, output a first target voltage (V_{T1}) to the load based on the received supply voltage (V_{SUP}). Accordingly, if the battery output voltage (V_{BAT}) is high enough, the first voltage regulator 81 outputs the supply voltage (V_{SUP}), which the second voltage regulator 82 regulates and provides a target voltage (V_{T1}) to the electrical load(s). In some embodiments, the second voltage regulator 82 may be configured to operate in a by-pass mode in response to receiving the supply voltage (V_{SUP}) from the first voltage regulator 81 at the input. Stated differently, the second voltage regulator 82 may simply forward the supply voltage (V_{SUP}) to the electrical loads, such that the first target voltage (V_{T1}) is the same as the supply voltage (V_{SUP}).

Further, the second voltage regulator 82 is configured to, in response to receiving the zero-voltage voltage from the first voltage regulator 81 at the input, output a second target voltage (V_{T2}) to the load by discharging the capacitor 83. In other words, if the first voltage regulator 81 is turned off by the undervoltage lockout functionality, the second voltage regulator 82 can at least temporarily supply a voltage to the electrical load(s) by discharging the capacitor 83. Stated differently, the second voltage regulator 82 may be configured to operate in a voltage regulation mode to regulate a voltage received from the capacitor 83 in response to receiving the zero-voltage voltage from the first voltage regulator 81 at the input.

The capacitor 83 may be a super capacitor. The capacitor 83 may have a capacitance in the range of 0.1F to 10F, such as for example 3F, 5F, or 8F. In particular, the capacitance of the capacitor may be chosen so that the second voltage regulator is capable of supplying the second target voltage (V_{T2}) to the electrical load(s) for a duration of 20s to 60s, such as for example 30s. This should bridge the initial current surge caused by activation of a source of negative pressure 14 or a valve 40, and mitigate the momentary voltage drop at the output of the battery 80.

The first target voltage (V_{T1}) and the second target voltage (V_{T2}) may be the same value. However, in some embodiments, the second target voltage (V_{T2}) is lower than the first target voltage (V_{T1}). By having a lower second target voltage (V_{T2}), the power supply arrangement 13 may be enabled to power the electrical load(s) for a longer duration of time or enable the use of smaller capacitors 83 while still achieving the advantageous functionality.

Furthermore, in some embodiments, the second voltage regulator 82 is configured to, in response to receiving the supply voltage (V_{SUP}) from the first voltage regulator 81 at the input, charge the capacitor 83. Accordingly, while the battery 80 outputs a sufficient voltage for the first voltage regulator 81 to operate, the second voltage regulator 82 can use some of that energy to charge the capacitor 83 for later use. In some embodiments, the capacitor 83 may be charged by a separate charge circuit (not shown) connected to the output of the first voltage regulator 81. The separate charge circuit may be an integrated component of the second voltage regulator 82.

An example of a suitable voltage regulator to be used as the first voltage regulator 81 referenced herein is the ISL91107 buck-boost regulator from Rensas^{®}, and an example of a suitable voltage regulator to be used as the second voltage regulator to be used as the second voltage regulator 82 referenced herein is the TPS61094 buck-boost regulator from Texas Instruments".

As mentioned, the first voltage regulator 81 may be provided with an UVLO functionality. In more detail, the power supply arrangement 13 may comprise an UVLO circuit 85 operatively connected to the first voltage regulator 81. The UVLO circuit 85 may be an integrated component of the first voltage regulator 81 or provided as a separate component. In more detail, the UVLO circuit 85 may be configured to monitor the input voltage (V_{BAT}), turn on the first voltage regulator 81 in response to the input voltage (V_{BAT}) being above the first voltage level (V₁), and turn off the first voltage regulator 81 in response to the input voltage (V_{BAT}) being below the second voltage level (V₂).

Accordingly, the UVLO circuit 85 monitors the input voltage (V_{BAT}) of the voltage regulator 81 through a voltage divider or similar sensing mechanism. Then, if the input voltage (V_{BAT}) exceeds a predefined upper threshold, the UVLO 85 circuit allows the regulator 81 to begin operating. This ensures the input voltage is high enough to power the regulator 81 and its load effectively. However, if the input voltage (V_{BAT}) drops below a predefined lower threshold, the UVLO 85 disables the regulator 81. This prevents the regulator 81 from operating at insufficient input voltage levels, which could lead to instability, malfunction, or damage.

Accordingly, the embodiments herein, reduce the need for prematurely replacing batteries in portable NPWT devices by mitigating the issue with UVLO caused by momentary voltage drops at the battery output at transient times with increased current demand from the components of the NPWT devices. Thereby improving sustainability. Moreover, the NPWT device according to some embodiments disclosed herein may achieve similar levels of operational stability with lower quality batteries as conventional NPWT devices may achieve with higher quality and more expensive batteries, thereby providing advantages in terms of cost efficiency.

Furthermore, some embodiments herein may improve patient safety. In more detail, NPWT devices may employ over-pressure protection (OVP) functionality, which is a function that monitors the pressure at the wound site 27 based on the output from the pressure sensor(s) 15 in order to protect the patient/user from discomfort caused by excess negative pressure being drawn. The purpose of the OVP functionality is to turn off the source of negative pressure 14 and potentially open the valve 40 if the pressure at the wound site 27 as reported by the pressure sensor(s) 15 is too low. However, just like any other component the OVP functionality needs operating power to be able to function, and by means of the embodiments disclosed herein, the chance of having the OVP functionality still operational, even when the battery output is too low is increased. Thus, an advantage of some embodiments is that the NPWT device may improve therapy and reduce the risk of patient discomfort.

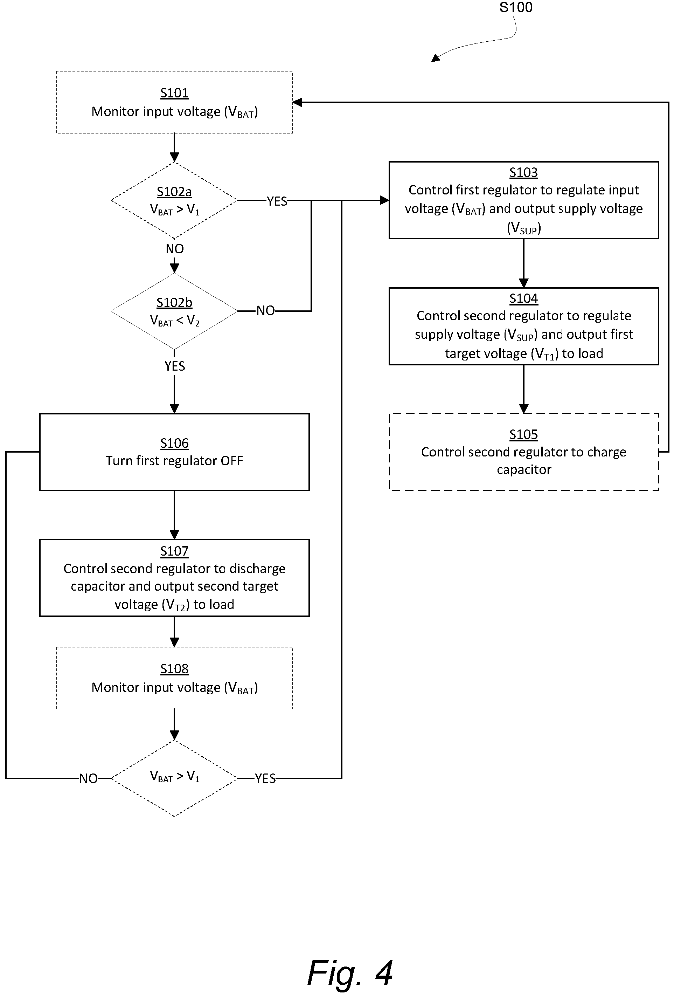

Fig. 4 is a schematic flowchart representation of a method for operating an apparatus 10 for providing negative pressure to a wound site 27. The apparatus 10 may be an apparatus according to any one of the embodiments disclosed herein. The method S100 is preferably a computer-implemented method S100, performed by a processing system of the apparatus 10. The processing system may for example comprise one or more processors 11 and one or more memories coupled to the one or more processors 11, wherein the one or more memories store one or more programs that perform the steps, services and functions of the method S100 disclosed herein when executed by the one or more processors.

The method S100 may comprise monitoring S101 an input voltage (V_{BAT}) as supplied from a battery of the apparatus. Further, in response to the input voltage (V_{BAT}) supplied from the battery being above a first voltage level, the method S100 may comprise outputting S103, using the first voltage regulator, a supply voltage (V_{SUP}) to the second voltage regulator based on the received input voltage (V_{BAT}). In other words, a first check S102a is performed to see if the supply voltage (V_{BAT}) is above a threshold (V₁), and if so, the first voltage regulator is activated S103. The method S100 further comprises outputting S104, using the second voltage regulator, a first target voltage (V_{T1}) to the load based on the received supply voltage (V_{SUP}). In other words, if the first voltage regulator is turned ON, then the second voltage regulator is controlled S104 to supply the specified voltage to the electrical load(s) of the apparatus. In this case, the second voltage regulator may be operated in a by-pass mode, simply forwarding the supply voltage (V_{SUP}) as its output voltage (V_{T1}). Moreover, in some embodiments, the method S100 may comprise controlling S105 the second voltage regulator to charge the capacitor.

However, if the input voltage (V_{BAT}) would be below a threshold, the first voltage regulator would be turned off (UVLO protection) and the second voltage regulator would be controlled to provide a target voltage by discharging the capacitor. Accordingly, in response to the input voltage (V_{BAT}) being below a second voltage level (V₂), the method S100 comprises outputting S106, using the first voltage regulator, a zero-voltage to the second voltage regulator, and outputting S107, using the second voltage regulator, a second target voltage (V_{T2}) to the electrical load by discharging the capacitor. As before, the first and second target voltages may be the same, or the second target voltage may be lower than the first target voltage.

Moreover, with respect to the threshold for the input voltage (V_{BAT}), the first and second voltage levels (V₁ and V₂) may be the same, or the second voltage level (V₂) may be lower than the first voltage level (V₁) in order to introduces a hysteresis functionality. In more detail, when the first voltage regulator is turned OFF due to a low input voltage (V_{BAT}) and the second voltage regulator supplies S107 power to the electrical loads by discharging the capacitor. The method S100 may comprise monitoring S108 the input voltage (V_{BAT}) during this operation. Then, in response to the input voltage (V_{BAT}) being above the first voltage level (V₁), the method S100 may comprise outputting S103, using the first voltage regulator, a supply voltage (V_{SUP}) to the second voltage regulator and outputting S104, using the second voltage regulator, a first target voltage (V_{T1}) to the electrical load. However, in response to the input voltage (V_{BAT}) being below the first voltage level (V₁), the method S100 comprises keeping the first voltage regulator turned OFF and outputting S107, using the second voltage regulator, a second target voltage (V_{T2}) to the electrical load by discharging the capacitor.

Executable instructions for performing these functions are, optionally, included in a non-transitory computer-readable storage medium or other computer program product configured for execution by one or more processors of an apparatus for providing reduced pressure to a wound dressing.

The herein disclosed technology has been presented above with reference to specific embodiments. However, other embodiments than the above described are possible and within the scope of the claims. Different method steps than those described above, performing the method by hardware or software, may be provided within the scope of the claims. Thus, according to some embodiments embodiment, there is provided a non-transitory computer-readable storage medium storing one or more programs configured to be executed by one or more processors of an apparatus of a reduced-pressure wound treatment system, the one or more programs comprising instructions for performing the method according to any one of the above-discussed embodiments.

Generally speaking, a computer-accessible medium may include any tangible or non-transitory storage media or memory media such as electronic, magnetic, or optical media coupled to computer system via bus. The terms "tangible" and "non-transitory," as used herein, are intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer-readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link.

The processor(s) 11 (associated with the apparatus 10) may be or include any number of hardware components for conducting data or signal processing or for executing computer code stored in memory. The device 10 may accordingly have an associated memory, and the memory may be one or more devices for storing data and/or computer code for completing or facilitating the various methods described in the present description. The memory may include volatile memory or non-volatile memory. The memory may include database components, object code components, script components, or any other type of information structure for supporting the various activities of the present description. According to some embodiments, any distributed or local memory device may be utilized with the systems and methods of this description. According to some embodiments embodiment the memory is communicably connected to the processor 11 (e.g., via a circuit or any other wired, wireless, or network connection) and includes computer code for executing one or more processes described herein.

It should be noted that any reference signs do not limit the scope of the claims, that some embodiments may be at least in part implemented by means of both hardware and software, and that several "means" or "units" may be represented by the same item of hardware.

Although the figures may show a specific order of method steps, the order of the steps may differ from what is depicted. In addition, two or more steps may be performed concurrently or with partial concurrence. Such variation will depend on the software and hardware systems chosen and on designer choice. All such variations are within the scope of the appended claims. Likewise, software implementations could be accomplished with standard programming techniques with rule-based logic and other logic to accomplish the generating steps, activating steps, operating steps, causing steps, steps. The above mentioned and described embodiments are only given as examples and should not be limiting to the appended claims. Other solutions, uses, objectives, and functions within the scope of the below described patent claims should be apparent for the person skilled in the art.

## Claims

1. An apparatus (10) for providing reduced pressure to a wound site (27), the apparatus comprising:
an electrical load;
a power supply arrangement (13) connected to the electrical load, the power supply arrangement (13) comprising:
a battery (80);
a first voltage regulator (81) connected to the battery;
a second voltage regulator (82) connected to the first voltage regulator and to the electrical load;
a capacitor (83) connected to the second voltage regulator;
wherein the first voltage regulator (81) is configured to receive an input voltage from the battery and to output a supply voltage to the second voltage regulator (82);
wherein the first voltage regulator (81) is further configured to:
in response to the input voltage being above a first voltage level:
output the supply voltage to the second voltage regulator (82) based on the received input voltage;
in response to the input voltage being below a second voltage level:
output a zero-voltage to the second voltage regulator (82);
wherein the second voltage regulator (82) is configured to:
in response to receiving the supply voltage from the first voltage regulator (81) at an input:
output a first target voltage to the electrical load based on the received supply voltage, and
in response to receiving the zero-voltage voltage from the first voltage regulator (81) at the input:
output a second target voltage to the electrical load by discharging the capacitor (83).

2. The apparatus (10) according to claim 1, wherein the capacitor (83) is a super capacitor.

3. The apparatus (10) according to claim 1 or 2, wherein the second voltage regulator (82) is configured to operate in a by-pass mode in response to receiving the supply voltage from the first voltage regulator at the input, and in a voltage regulation mode to regulate a voltage received from the capacitor in response to receiving the zero-voltage voltage from the first voltage regulator (81) at the input.

4. The apparatus (10) according to any one of claims 1-3, wherein the first voltage regulator (81) is configured to operate in a voltage regulation mode in order to regulate the input voltage in response to the input voltage being above the first voltage level, and in a undervoltage lockout mode in order to turn off the first voltage regulator (81) in response to the input voltage being below the second voltage level.

5. The apparatus (10) according to any one of claims 1-4, wherein the electrical load comprises at least one of:
a source of negative pressure (14) configured to provide negative pressure via a first fluid flow path from an inlet of the source of negative pressure to the wound site;
an electronically controllable valve (40) configured to release negative pressure from the wound site via a second fluid flow path;
control circuitry (11); or
a loudspeaker (84) configured to output sound to the user of the apparatus (10).

6. The apparatus (10) according to any one of claims 1-5, wherein the first voltage level is the same as the second voltage level.

7. The apparatus (10) according to any one of claims 1-5, wherein the first voltage level is higher than the second voltage level.

8. The apparatus (10) according to any one of claims 1-7, wherein the power supply arrangement (13) comprises:
an undervoltage-lockout circuit (85) operatively connected to the first voltage regulator (81), wherein the under-voltage lockout circuit (85) is configured to:
monitor the input voltage;
turn on the first voltage regulator (81) in response to the input voltage being above the first voltage level; and
turn off the first voltage regulator (81) in response to the input voltage being below the second voltage level.

9. The apparatus (10) according to any one of claims 1-8, wherein the second voltage regulator (82) is configured to:
in response to receiving the supply voltage from the first voltage regulator (81) at an input:
charge the capacitor (83).

10. The apparatus (10) according to any one of claims 1-9, wherein the first target voltage is higher than the second target voltage.

11. A system comprising:
an apparatus (10) according to any one of claims 1-10;
a wound cover (22) for creating a sealed space defined in part by the wound site (27); and
a tubing assembly defining the fluid flow path.

12. A computer-implemented method (S100) for operating an apparatus (10) for providing negative pressure to a wound site, wherein the apparatus comprises an electrical load and a power supply arrangement connected to the electrical load, wherein the power supply arrangement comprises a battery, a first voltage regulator, a second voltage regulator, and a capacitor connected to the second voltage regulator, the computer-implemented method comprising:
in response to an input voltage supplied from the battery being above a first voltage level:
outputting (S103), using the first voltage regulator, a supply voltage to the second voltage regulator based on the received input voltage,
outputting (S104), using the second voltage regulator, a first target voltage to the electrical load based on the received supply voltage;
in response to the input voltage being below a second voltage level:
outputting (S106), using the first voltage regulator, a zero-voltage to the second voltage regulator,
outputting (S107), using the second voltage regulator, a second target voltage to the electrical load by discharging the capacitor.

13. The computer-implemented method (S100) according to claim 12, wherein the electrical load comprises at least one of:
a source of negative pressure (14) configured to provide negative pressure via a first fluid flow path from an inlet of the source of negative pressure to the wound site;
an electronically controllable valve (40) configured to release negative pressure from the wound site via a second fluid flow path;
control circuitry; or
a loudspeaker configured to output sound to the user of the apparatus (10).

14. The computer-implemented method (S100) according to claim 12 or 13, wherein the first voltage level is higher than the second voltage level.

15. A computer program product comprising instructions which, when the program is executed by a computing device of an apparatus for providing negative pressure to a wound site, causes the apparatus to carry out the method (S100) according to any one of claims 12-14
